# EUROPÄISCHE PATENTSCHRIFT

(11) **EP 0 537 591 B1**
(45) Veröffentlichungstag und Bekanntmachung des Hinweises auf die Patenterteilung: **27.09.1995**
(21) Anmeldenummer: 92117008.0
(22) Anmeldetag: 06.10.1992
(51) Int. Cl.: C07C 205/37, C07C 201/12

(54) **Verfahren zur Herstellung von 1,2-Bis-(2-nitrophenoxy)-ethan**
Process for the preparation of 1,2-bis-(2-nitrophenoxy)-ethane
Procédé pour la préparation de 1,2-bis-(2-nitrophénoxy)-éthane

(30) Priorität: 09.10.1991 DE 4133446
(43) Veröffentlichungstag der Anmeldung: 21.04.1993
(73) Patentinhaber: HOECHST AKTIENGESELLSCHAFT, 65926 Frankfurt am Main (DE)
(72) Erfinder: Papenfuhs, Theodor, Dr., W-6000 Frankfurt/Main (DE); Pressler, Wilfried, Dr., W-6233 Kelkheim/Ts (DE); Rapp, Jochen, Dr., W-6000 Frankfurt am Main (DE)

(56) Entgegenhaltungen:
- EP-A- 0 200 955
- FR-A- 2 319 610

## Beschreibung

Die Erfindung betrifft ein verbessertes Verfahren zur Herstellung von 1,2-Bis-(2-nitrophenoxy)-ethan in hoher Ausbeute und sehr guter technischer Reinheit durch Veretherung von Ethylenglykol mit 2-Chlornitrobenzol, wobei zur Veretherung in N,N-Dimethylacetamid als Lösungsmittel gearbeitet wird und die zur Veretherung benötigte Base fest oder als Suspension in das Dimethylacetamid eingetragen wird. Das so erhältliche Produkt kann ohne Reinigung zum 1,2-Bis-(2-aminophenoxy)-ethan reduziert und weiterverarbeitet werden.

1,2-Bis-(2-nitrophenoxy)-ethan kann als Fungizid eingesetzt werden (JP 48-010527). Ebenso ist 1,2-Bis-(2-nitrophenoxy)-ethan ein wichtiges Zwischenprodukt bei der Herstellung von Pigmenten. So führt Reduktion zum entsprechenden Diamin, dessen Tetrazotierung und anschließende Kupplung mit 5-Acetoacetylamino-benzimidazolonen zu wertvollen Gelbpigmenten (EP-PS 0 024 702).

Es ist bekannt, 1,2-Bis-(2-nitrophenoxy)-ethan durch Umsetzung von Kalium-2-nitrophenolat mit 1,2-Dichlorethan oder 1,2-Dibromethan in Alkohol (A.C. Cope, J. Am. Chem. Soc. 57 [1935] 572) oder von Natrium-2-nitrophenolat mit 1,2-Dichlorethan (A. Weddige, J. Prakt. Chem 21 [1880] 127) oder mit 1,2-Dibromethan in Alkohol (R. Jaunin, R. Holl, Helv. Chim. Acta 41 [1958] 1783, 1789) herstellen zu können.

Die Übertragung dieser bekannten Verfahren in technischen Maßstab ist unter heutigen Gesichtspunkten nicht möglich. So ist die Veretherung mit 1,2-Dibromethan aufgrund der hohen Kosten für 1,2-Dibromethan unwirtschaftlich.

Ferner ist 1,2-Dibromethan ebenso wie 1,2-Dichlorethan, in höchstem Maße gesundheitsschädlich, so daß diese Verbindung aus Gründen der Arbeitshygiene in einem technischen Verfahren unbedingt zu vermeiden ist. Weiterhin sind ohne apparative Aufwendungen Emissionen der Ethylenhalogenide, die bereits bei Raumtemperatur sehr hohen Dampfdruck besitzen, nicht zu vermeiden. Prohibitiv für die Umsetzung in technischem Maßstab ist auch die Bildung von karzinogenen Nebenprodukten, wie Vinylchlorid und Vinylbromid, die bei den vorstehend genannten Reaktionsbedingungen der bekannten Verfahren auftreten. Schließlich sind die genannten bekannten Verfahren auch wegen der Ausbeuten (zwischen 30 und 85 %) unwirtschaftlich.

Als bekannte Alternative zu den vorstehend genannten bekannten Verfahren zur Herstellung von 1,2-Bis-(2-nitrophenoxy)-ethan kann man 63 Teile Ethylenglykol mit 315 Teilen 2-Chlornitrobenzol und 480 Teilen einer 50 %igen, wäßrigen Natriumhydroxydlösung in Gegenwart von 80 Teilen einer 50 %igen, wäßrigen Lösung von Benzyl-dimethyl-laurylammoniumchlorid als Phasentransferkatalysator bei 90°C umsetzen (DE 26 34 419 A1). Die tropfenweise Zugabe der Natriumhydroxydlösung dauert hierbei 3 Stunden und die Nachreaktion erfordert 16 Stunden, wodurch die Wirtschaftlichkeit des Verfahrens stark vermindert wird. Das Reaktionsgemisch wird zur Ausfällung des Produkts in 750 Teile Wasser gegeben und gesammelt.

Unvorteilhaft bei diesem Verfahren ist die Reinigung des erhaltenen Rohprodukts. Der Filterkuchen muß in 500 Teilen Wasser aufgeschlämmt und mit Salzsäure auf pH 3-4 gestellt werden. Der Feststoff wird erneut gesammelt und abschließend mit zunächst 350 Teilen, dann mit 80 Teilen Aceton gewaschen und getrocknet.

Nachteilig an der genannten Herstellungsvariante ist zum einen der erforderliche Überschuß von 400 mol-% (bezogen auf eingesetztes Ethylenglykol) an Natriumhydroxyd, zum anderen und viel schwerwiegender die Tatsache, daß ein stark salzhaltiges, hochgradig alkalisches, mit 2-Nitrophenol (Nebenprodukt) und Phasentransferkatalysator, der nicht rückführbar ist, belastetes Abwasser anfällt.

Es bestand somit ein Bedarf für ein verbessertes, technisches Verfahren zur Herstellung von 1,2-Bis-(2-nitrophenoxy)-ethan, das in einer Verfahrensstufe unter Vermeidung der vorstehend beschriebenen Nachteile oder bekannten Verfahren in guter Ausbeute und hoher Reinheit zu möglichst durch einfache Filtration abtrennbarem Produkt führt.

Es wurde nun überraschenderweise gefunden, daß man 1,2-Bis-(2-nitrophenoxy)-ethan in sehr guter Ausbeute und hoher Reinheit in einem Einstufenverfahren herstellen kann, indem man 1 Mol Ethylenglykol mit 190 bis 260 mol-%, vorzugsweise mit 200 bis 250 mol-%, besonders bevorzugt mit 210 bis 220 mol-% 2-Chlornitrobenzol bei Temperaturen von 40 bis 100°C, vorzugsweise von 50 bis 80°C, besonders bevorzugt von 55 bis 65°C, in Gegenwart eines Alkalimetallhydroxids in Dimethylacetamid (nachstehend "DMAc" genannt) umsetzt.

Das Alkalimetallhydroxid wird zweckmäßigerweise in einer Menge von 200 bis 300 mol-%, bevorzugt von 230 bis 260 mol-%, besonders bevorzugt von 240 bis 250 mol-%, bezogen auf Ethylenglykol, eingesetzt. Wenn auch prinzipiell alle Alkalimetallhydroxide, wie Lithium-, Natrium-, Kalium-, Rubidium- oder Cäsiumhydroxyid sowie Mischungen daraus angewandt werden können, so werden doch vorzugsweise Natrium- und/oder Kaliumhydroxid eingesetzt. Die Alkalimetallhydroxide, wie vorzugsweise das Kalium- oder Natriumhydroxid, werden entweder in fester Form oder suspendiert in DMAc kontinuierlich oder in Anteilen in die Reaktionsmischung eingetragen. Besonders bevorzugt ist die Anwendung einer Suspension von Natriumhydroxid in DMAc.

Das Eintragen des Alkalimetallhydroxids benötigt in der Regel zwischen 2 und 4 Stunden. Nach beendeter Zugabe wird noch etwa 2 bis 3 Stunden nachgerührt.

Anschließend wird bei der gewählten Reaktionstemperatur unter Zuhilfenahme einer pH-Elektrode die Reaktionsmischung mit einer konzentrierten Mineralsäure auf einen pH-Wert von 7,0 bis 5,5, vorzugsweise von etwa 6,5 bis 6,0 gestellt. Geeignete Mineralsäuren sind beispielsweise höher konzentrierte Salz-, Bromwasserstoff-, Jodwasserstoff-, Schwefel- oder Phosphorsäure. Von der heißen Reaktionsmischung werden die anorganischen Salze abfiltriert und mit DMAc gewaschen. Im Filtrat wird durch Zusatz von Wasser oder niederen aliphatischen Alkoholen das angefallene Produkt ausgefällt. Es wird gesammelt und mit Wasser oder Alkohol gewaschen. Vom Filtrat werden das Wasser oder der Alkohol und DMAc destillativ zurückgewonnen und in den Produktionsprozeß recyclisiert.

Das erfindungsgemäße Verfahren wird zweckmäßigerweise bei Atmosphärendruck durchgeführt; es kann jedoch auch bei erhöhtem oder vermindertem Druck (Vakuum) vorgenommen werden.

Durch das nachstehende Beispiel wird das erfindungsgemäße Verfahren näher erläutert, ohne darauf beschränkt zu werden.

### Beispiel

In einem 2l Witt'schen Topf mit Rührer, Strömungsbrecher und Innenthermometer werden 700 Teile DMAc, 362 Teile 2-Chlornitrobenzol und 68 Teile Ethylenglykol vorgelegt und auf 55°C aufgeheizt. Dann wird bei 55°C eine Suspension von 108 Teilen Natriumhydroxyd in 100 Teilen DMAc während 4 Stunden so zugepumpt, daß die Reaktionstemperatur 60°C nicht übersteigt. Darauf wird 2,5 Stunden bei 60°C nachgerührt. Dann wird die Reaktionsmischung mit 69 Teilen 30 %iger Salzsäure auf pH 6,5 getellt. Nach Erhitzen auf 110°C wird von der Reaktionsmischung Salz abgesaugt und dieses mit 100 Teilen DMAc gewaschen. Zur Produktkristallisation werden bei 90°C 300 Teile Wasser in das Filtrat eingerührt. Es wird auf 10°C gekühlt und das 1,2-Bis-(2-nitritphenoxy)-ethan abgesaugt und mit 100 Teilen Wasser gewaschen. Wasser und DMAc werden aus dem Filtrat destillativ zurückgewonnen und in den Produktionsprozeß recyclisiert. Das wasserfeuchte hellbeige, kristalline Produkt kann bei Bedarf getrocknet werden. Das getrocknete Produkt fällt mit einem Schmelzpunkt von 167-168°C in einer Ausbeute von 93 % d.Th. mit einer Reinheit von 98,5 % nach HPLC an.

Verwendet man anstelle der 108 Teile Natriumhydroxid 151 Teile Kaliumhydroxid in 100 Teilen DMAc und säuert man die Reaktionsmischung statt mit 69 Teilen 30 %iger Salzsäure mit 58 Teilen 96%iger Schwefelsäure auf pH 6,5 an, so gelangt man zum gleichen Resultat.

## Patentansprüche

1. Verfahren zur Herstellung von 1,2-Bis-(2-nitrophenoxy)-ethan, dadurch gekennzeichnet, daß man 1 Mol Ethylenglykol mit 190 bis 260 mol-% 2-Chlornitrobenzol bei Temperaturen von 40 bis 100°C unter Zusatz eines Alkalimetallhydroxids in Dimethylacetamid umsetzt.

2. Verfahren nach Anspruch 1, dadurch gekennzeichnet, daß man bei Temperaturen von 50 bis 80°C umsetzt.

3. Verfahren nach mindestens einem der Ansprüche 1 und 2, dadurch gekennzeichnet, daß man bei Temperaturen von 55 bis 65°C umsetzt.

4. Verfahren nach mindestens einem der Ansprüche 1 bis 3, dadurch gekennzeichnet, daß man das Alkalimetallhydroxid der Reaktionsmischung in fester Form zusetzt.

5. Verfahren nach mindestens einem der ansprüche 1 bis 3, dadurch gekennzeichnet, daß man das Alkalimetallhydroxid der Reaktionsmischung suspendiert in Dimethylacetamid zusetzt.

6. Verfahren nach mindestens einem der Ansprüche 1 bis 4, dadurch gekennzeichnet, daß man unter Zusatz von festem Natriumhydroxid oder Kaliumhydroxid oder Mischungen daraus umsetzt.

7. Verfahren nach mindestens einem der Ansprüche 1 bis 3 und 5, dadurch gekennzeichnet, daß man unter Zusatz von in Dimethylacetamid suspendiertem Natriumhydroxid oder Kaliumhydroxid oder Mischungen daraus umsetzt.

8. Verfahren nach mindestens einem der Ansprüche 1 bis 7, dadurch gekennzeichnet, daß man mit 200 bis 250 mol-% 2-Chlornitrobenzol umsetzt.

9. Verfahren nach mindestens einem der Ansprüche 1 bis 8, dadurch gekennzeichnet, daß man mit 210 bis 220 mol-% 2-Chlornitrobenzol, umsetzt.

10. Verfahren nach mindestens einem der Ansprüche 1 bis 9, dadurch gekennzeichnet, daß man unter Zusatz von 200 bis 300 mol-% Alkalimetallhydroxid, bezogen auf Ethylenglykol, umsetzt.

11. Verfahren nach mindestens einem der Ansprüche 1 bis 10, dadurch gekennzeichnet, daß man unter Zusatz von 230 bis 260 mol-% Alkalimetallhydroxid, bezogen auf Ethylenglykol, umsetzt.

12. Verfahren nach mindestens einem der Ansprüche 1 bis 11, dadurch gekennzeichnet, daß man unter Zusatz von 240 bis 250 mol-% Alkalimetallhydroxid, bezogen auf Ethylenglykol, umsetzt.

13. Verfahren nach mindestens einem der Ansprüche 1 bis 3,5 und 7 bis 12, dadurch gekennzeichnet, daß man in Dimethylacetamid suspendiertes Alkalimetallhydroxid der Reaktionsmischung kontinuierlich oder in Anteilen zusetzt.

14. Verfahren nach mindestens einem der Ansprüche 1 bis 13, dadurch gekennzeichnet, daß man die ausreagierte Reaktionsmischung mit konz. Salzsäure oder Schwefelsäure auf einen pH-Wert von 6,5 bis 6,0 stellt.

15. Verfahren nach mindestens einem der Ansprüche 1 bis 14, dadurch gekennzeichnet, daß man das Reaktionsprodukt durch Zusatz von Wasser oder eines niederen aliphatischen Alkohols ausfällt.

16. Verfahren nach mindestens einem der Ansprüche 1 bis 13, dadurch gekennzeichnet, daß man bei Atmosphärendruck oder einem erhöhten oder erniedrigten Druck (Vakuum) umsetzt.

## Claims

1. A process for the preparation of 1,2-bis-(2-nitrophenoxy)-ethane, which comprises reacting 1 mole of ethylene glycol with 190 to 260 mol% of 2-chloronitrobenzene at temperatures of 40 to 100°C, with the addition of an alkali metal hydroxide, in dimethylacetamide.

2. The process as claimed in claim 1, wherein the reaction is carried out at temperatures of 50 to 80°C.

3. The process as claimed in at least one of claims 1 and 2, wherein the reaction is carried out at temperatures of 55 to 65°C.

4. The process as claimed in at least one of claims 1 to 3, wherein the alkali metal hydroxide is added to the reaction mixture as a solid.

5. The process as claimed in at least one of claims 1 to 3, wherein the alkali metal hydroxide is added to the reaction mixture as a suspension in dimethylacetamide.

6. The process as claimed in at least one of claims 1 to 4, wherein the reaction is carried out with the addition of solid sodium hydroxide or potassium hydroxide or mixtures thereof.

7. The process as claimed in at least one of claims 1 to 3 and 5, wherein the reaction is carried out with the addition of sodium hydroxide or potassium hydroxide or mixtures thereof suspended in dimethylacetamide.

8. The process as claimed in at least one of claims 1 to 7, wherein the reaction is carried out with 200 to 250 mol% of 2-chloronitrobenzene.

9. The process as claimed in at least one of claims 1 to 8, wherein the reaction is carried out with 210 to 220 mol% of 2-chloronitrobenzene.

10. The process as claimed in at least one of claims 1 to 9, wherein the reaction is carried out with the addition of 200 to 300 mol% of alkali metal hydroxide, relative to ethylene glycol.

11. The process as claimed in at least one of claims 1 to 10, wherein the reaction is carried out with the addition of 230 to 260 mol% of alkali metal hydroxide, relative to ethylene glycol.

12. The process as claimed in at least one of claims 1 to 11, wherein the reaction is carried out with the addition of 240 to 250 mol% of alkali metal hydroxide, relative to ethylene glycol.

13. The process as claimed in at least one of claims 1 to 3, 5 and 7 to 12, wherein alkali metal hydroxide suspended in dimethylacetamide is added to the reaction mixture continuously or in portions.

14. The process as claimed in at least one of claims 1 to 13, wherein the fully reacted reaction mixture is adjusted to a pH of 6.5 to 6.0 with concentrated hydrochloric acid or sulfuric acid.

15. The process as claimed in at least one of claims 1 to 14, wherein the reaction product is precipitated by the addition of water or a lower aliphatic alcohol.

16. The process as claimed in at least one of claims 1 to 13, wherein the reaction is carried out at atmospheric pressure or an elevated or reduced pressure (vacuum).

## Revendications

1. Procédé de préparation du 1,2-bis-(2-nitrophénoxy)-éthane, caractérisé en ce qu'on met en réaction 1 mole d'éthylèneglycol avec de 190 à 260 % en mole de 2-chloronitrobenzène à des températures comprises entre 40 et 100°C en additionnant un hydroxyde de métal alcalin dans du diméthylacétamide.

2. Procédé selon la revendication 1, caractérisé en ce qu'on effectue la réaction à des températures comprises entre 50 et 80°C.

3. Procédé selon au moins l'une des revendications 1 et 2, caractérisé en ce qu'on effectue la réaction à des températures comprises entre 55 et 65°C.

4. Procédé selon au moins l'une des revendications 1 à 3, caractérisé en ce qu'on ajoute l'hydroxyde de métal alcalin au mélange réactionnel sous la forme solide.

5. Procédé selon au moins l'une des revendications 1 à 3, caractérisé en ce qu'on ajoute l'hydroxyde de métal alcalin au mélange réactionnel en suspension dans du diméthylacétamide.

6. Procédé selon au moins l'une des revendications 1 à 4, caractérisé en ce qu'on effectue la réaction en additionnant de l'hydroxyde de potassium ou de l'hydroxyde de sodium solide ou l'un de leurs mélanges.

7. Procédé selon au moins l'une des revendications 1 à 3 et 5, caractérisé en ce qu'on effectue la réaction en additionnant de l'hydroxyde de potassium ou de l'hydroxyde de sodium ou bien l'un de leurs mélanges en suspension dans du diméthylacétamide

8. Procédé selon au moins l'une des revendications 1 à 7, caractérisé en ce qu'on effectue la réaction avec de 200 à 250 % en mole de 2-chloronitrobenzène.

9. Procédé selon au moins l'une des revendications 1 à 8, caractérisé en ce qu'on effectue la réaction avec de 210 à 220 % en mole de 2-chloronitrobenzène.

10. Procédé selon au moins l'une des revendications 1 à 9, caractérisé en ce qu'on effectue la réaction en additionnant de 200 à 300 % en mole d'hydroxyde de métal alcalin, par rapport à l'éthylèneglycol.

11. Procédé selon au moins l'une des revendications 1 à 10, caractérisé en ce qu'on effectue la réaction en additionnant de 230 à 260 % en mole d'hydroxyde de métal alcalin, par rapport à l'éthylèneglycol.

12. Procédé selon au moins l'une des revendications 1 à 11, caractérisé en ce qu'on effectue la réaction en additionnant de 240 à 250 % en mole d'hydroxyde de métal alcalin, par rapport à l'éthylèneglycol.

13. Procédé selon au moins l'une des revendications 1 à 3, 5 et de 7 à 12, caractérisé en ce qu'on ajoute l'hydroxyde de métal alcalin au mélange réactionnel en suspension dans du diméthylalcétamide en continu ou par portion.

14. Procédé selon au moins l'une des revendications 1 à 13, caractérisé en ce qu'on place le mélange réactionnel ayant réagi à un pH compris entre 6,5 et 6,0 avec de l'acide sulfurique ou de l'acide chlorhydrique concentré.

15. Procédé selon au moins l'une des revendications 1 à 14, caractérisé en ce qu'on fait précipiter le produit de réaction en additionnant de l'eau ou un alcool aliphatique inférieur.

16. Procédé selon au moins l'une des revendications 1 à 13, caractérisé en ce qu'on effectue la réaction à pression atmosphérique ou à une pression élevée ou inférieure (sous vide).
